# EUROPÄISCHE PATENTSCHRIFT

(11) **EP 1 190 024 B1**
(45) Veröffentlichungstag und Bekanntmachung des Hinweises auf die Patenterteilung: **11.09.2002**
(21) Anmeldenummer: 00958353.5
(22) Anmeldetag: 02.08.2000
(51) Int. Cl.: C11B 1/06, C11B 1/02, A21D 2/26, A23K 1/14

(54) **TRAUBENKERNE, KALTGEPRESSTES TRAUBENKERNÖL, TRAUBENKERNSCHROT UND TRAUBENKERNMEHL**
GRAPESEED, COLD-PRESSED GRAPE OIL, CRUSHED GRAPE AND GRAPE FLOUR
PEPINS DE RAISIN, HUILE DE PEPINS DE RAISIN PRESSEE A FROID, ENVELOPPE DE PEPINS DE RAISIN ET FARINE DE PEPINS DE RAISIN

(30) Priorität: 05.08.1999 DE 19936492; 25.03.2000 DE 10015006
(43) Veröffentlichungstag der Anmeldung: 27.03.2002
(73) Patentinhaber: Vitis Ölmühlen KG, 54349 Trittenheim (DE)
(72) Erfinder: ECKERT, Peter, D-53127 Bonn (DE); HEINEN, Winfrid, D-54340 Leiwen (DE); KNAUDT, Carola, D-53125 Bonn (DE)
(74) Vertreter: Helbing, Jörg, Dr. Dipl.-Chem.
(86) Internationale Anmeldenummer: EP0007460
(87) Internationale Veröffentlichungsnummer: WO01010987

(56) Entgegenhaltungen:
- BE-A- 875 360
- DD-A- 217 999
- FR-A- 2 764 776
- ROHNE. G: "Extraccion del aceite de la granilla de uva" GRASAS Y ACEITES, Bd. 22, Nr. 5, 1971, Seiten 393-400, XP000971139 SEVILLA, ES
- PALMA M ET AL: "Extraction of polyphenolic compounds from grape seeds with near critical carbon dioxide" JOURNAL OF CHROMATOGRAPHY A,NL,ELSEVIER SCIENCE, Bd. 849, Nr. 1, 16. Juli 1999 (1999-07-16), Seiten 117-124, XP004173682 ISSN: 0021-9673
- DATABASE WPI Week 8903 Derwent Publications Ltd., London, GB; AN 1989-017755 XP002155297 & HU 3 201 A (SZEKSZARDI BORASZATI KOESZOES V), 28. November 1988 (1988-11-28)

## Beschreibung

Die vorliegende Anmeldung betrifft ein Verfahren zur Herstellung von kaltgepresstem Traubenkernöl sowie Nahrungsmittel, Nahrungsergänzungsmittel, Nahrungszusatzfaktoren, Genussmittel, Tiernahrungsmittel, Tiernahrungsergänzungsfaktoren, Arzneimittel und Kosmetika/Hautpflegemittel, umfassend das gemäß dem Verfahren hergestellte kaltgepresste Traubenkernöl, ebenso die Anreicherung der vorgenannten Stoffe/Mittel und gleichermaßen die Anreicherung von Wein und anderen Getränken, kaltgepresstem Traubenkernöl und anderen Ölen mit cyclischen Polyphenolen und anderen Wirkstoffen, die aus dem bei dem Verfahren anfallenden Kernschrot extrahiert werden können. Die Anmeldung betrifft auch das gemäß dem Verfahren hergestellte kaltgepresste Traubenkernöl, den bei diesem Verfahren anfallenden Kernschrot und Gemische derselben.

Schon im Mittelalter war bekannt, dass aus Traubenkernen durch kalte Pressung hochwertiges Speiseöl gewonnen werden kann. Dieses Öl wurde weiterhin als wirksames Mittel in der Kosmetik und als Mittel zur Behandlung von kleineren Wunden wie Verbrennungen und Hautabschürfungen und zur Behandlung von rissiger Haut eingesetzt (W. Heinen, in "Das deutsche Weinmagazin", 14, Juli 1997). Das althergebrachte Verfahren des Kaltpressens der Weintraubenkerne geriet vollständig in Vergessenheit und wurde durch moderne Extraktionsverfahren ersetzt.

Es ist bekannt, dass Traubenkerne einen extrem hohen - sehr spezifischen - Gehalt an zyklischen Polyphenolen wie Procyanidine (Epigallocatechin-3-gallate), z. B. das Catechin, sein diastereomers Epicatechin, die Procyanidine B1, B2 und B3 sowie Procyanidin C1 und diverse Proanthocyanidine P1-P2 besitzen. All diesen natürlichen Verbindungen, subsummiert als Flavanole (enthalten einen Hydroxyphenylrest) werden seit Jahrzehnten aufgrund ihrer herausragender antioxidativen Potenz eine hohe biologische Aktivität zugemessen.

Wie jüngste Untersuchungen zeigen, hemmen cyclische Polyphenole wichtige Rezeptoren der menschlichen Zellmembran, die den 1-Helix-Oberflächenrezeptoren zuzurechnen sind und zur Bildung und Steuerung von verschiedenen Wachstumshormonen, aber auch dem Insulin beitragen. Dieser Rezeptortyp wird von Proteinkirlasen gebildet, die ihrerseits eine zentrale Aufgabe bei der intrazellulären Signalvermittlung einnehmen. Sie bewirken durch Phosphorylierung von Proteinen eine Veränderung der biologischen Aktivität. Hauptvertreter sind die Tyrosinkinasen, die aktiv bei der Over- und Down-Regulierung des Epithel Growth Factors (EGF) des Platelet Derived Groth Factor (PDGF), des Fibroblasten Growth Factor (FGF_{F}) und des Insulinrezeptors mitwirken. Diese Rezeptoren können durch "Überfunktion", Overexpression, ihre Zelle schädigen und eine bestimmte Krankheit herbeiführen. Mit der Aktivierung und Hemmung der Tyrosinkinasen sind Krankheiten wie Arteriosklerose, arterielle Hypertonie, Wachstum und Ausbreitung diverser Tumore, Hautkrankheiten und Insulinregulation verbunden.

Überraschenderweise wurde gefunden, dass der Anteil der biologisch wirksamen Verbindungen, insbesondere der Polyphenolverbindungen in Traubenkernölen äußerst abhängig ist von der Art des Pressverfahrens, insbesondere der Presstemperatur und dem Grad der Verunreinigung des Ausgangskernmaterials. So ist der Gehalt von solchen Polyphenolen in durch Heißextraktionsverfahren gewonnene Traubenkernöl deutlich geringer als in einem herkömmlichen, "klassischen" Kaltpressverfahren, wie es von W. Heinen in "Das deutsche Weinmagazin", 14, Juli 1997, beschrieben wurde. Auch führt die analoge Anwendung des in DD-A-217999 beschriebenen Verfahrens zu einem geringen Polyphenolgehalt in Traubenkernöl.

Es konnte nun gefunden werden, dass durch eine sorgfältige Reinigung des Pressgutes von Traubenhäuten, Stielen, Stengeln etc. wie auch durch eine Abtrennung von Metall- (d. h. Eisen-) wie auch durch die Kontrolle von Pressdruck und Presstemperatur andererseits, der Gehalt an cyclischen Polyphenolen des kaltgepressten Traubenkernöls verbessert werden kann. Ebenso wurde gefunden, dass der verbleibende Kernschrot außerordentlich hohe Gehalte an cyclischen Polyphenolen hat und Auszüge dieser Stoffe aus dem Mehl des Kernschrotes in Öl, Wein und anderen Flüssigkeiten diese mit den genannten Wertstoffen in hohem Maße anreichern.

Weiterhin wurde gefunden, dass die Reinigung des kaltgepressten Traubenkernöls möglichst durch Sedimentation erfolgen sollte, da hierbei die in dem kaltgepressten Traubenkernöl vorhandenen Wirksubstanzen am besten erhalten werden können.

Es konnte gezeigt werden, dass durch eine dem Pressverfahren nachfolgende Lagerung des Traubenkernöles auf seinem Kernschrotmehl ohne Hilfsmittel, ohne mechanische oder chemische Einwirkung der Gehalt an Polyphenolverbindungen im Traubenkernöl auf natürliche Weise erhöht werden kann. Weiterhin wurde gefunden, dass sowohl das so hergestellte Traubenkernöl wie auch der in dem Verfahren erhältliche Kernschrot sowie ein Gemisch aus beiden ein äußerst effektives Nahrungsmittel bzw. Nahrungsmittelergänzung für Mensch und Tier, ein wirksames Arzneimittel und wirksamer Bestandteil von Kosmetika ist. Hierbei ist insbesondere die Verwendung zur Behandlung von Hauterkrankungen zu erwähnen.

Gegenstand der vorliegenden Anmeldung ist somit
(1) ein Verfahren zum Gewinnen und Verarbeiten von hoch gereinigten Traubenkernen, umfassend die Schritte
   (a) schonendes Trocknen der Traubenkerne und
   (b) Reinigen der getrockneten Traubenkerne, das Sieben und Abtrennung von Metallteilen mittels Dauermagneten umfasst,
(2) ein Verfahren zur Herstellung von kaltgepresstem Traubenkernöl (nachfolgend auch "Traubenkernöl" und "KTK"), umfassend Trocknen und Reinigen der Traubenkerne, wie vorstehend unter (1) beschrieben und
   (c) Pressen der gereinigten Traubenkerne bei einem Pressdruck im Presskopf von 50 bis 100 kN unter Erhalt von kaltgepresstem Traubenkernöl und Kernschrot (nachfolgend auch "KS");
(3) eine besondere Ausführungsform des vorstehend unter (2) beschriebenen Verfahrens, weiterhin umfassend die Schritte
   (d) Extraktion von Inhaltstoffen aus dem gemäß dem unter (2) beschriebenen Verfahren erhältlichen Kernschrot durch Versetzen des Kernschrots mit dem gemäß dem unter (2) beschriebenen Verfahren erhältlichen kaltgepressten Traubenkernöl, ebenso mit anderen Speiseölen,
   (e) Stehenlassen des in Schritt (d) erhältlichen Gemisches und
   (f) Abtrennen des Traubenkernöls von dem Kernschrot;
(4) Nahrungsmittel, Nahrungsergänzungsmittel, Tiernahrungsmittel, Arzneimittel und Kosmetika in allen Darreichungsformen, umfassend
   (a) das gemäß dem unter (1) bis (3) beschriebenen, insbesondere das gemäß dem unter (3) beschriebenen Verfahren erhältliche kaltgepresste Traubenkernöl,
   (b) den gemäß dem unter (1) und (2) beschriebenen Verfahren erhältlichen Kernschrot oder
   (c) ein Gemisch aus (a) und (b),
(5) Verfahren zur Extraktion von Inhaltsstoffen aus dem gemäß dem unter (2) beschriebenen Verfahren erhältlichen Kernschrot, umfassend das Versetzen des Kernschrots mit einem geeigneten Extraktionsmittel wie Ölen, Weinen (sowohl Weiß-, Rose- und Rotwein) und anderen alkoholischen und nichtalkoholischen Getränken sowie anderen Flüssigkeiten;
(6) Nahrungsmittel und Nahrungsergänzungsmittel, enthaltend die gemäß dem unter (1) beschriebenen Verfahren erhältlichen Traubenkerne und/oder Traubenkemmehl aus den genannten Traubenkernen und
(7) Verwendung des in (4) definierten Gemisches zur Behandlung von Hauterkrankungen, insbesondere Psoriasis.

Die vorliegende Erfindung wird nachfolgend unter Bezugnahme auf die Zeichnungen näher beschrieben:

Es zeigen:
Fig. 1 eine erfindungsgemäße Vorrichtung zur Reinigung von Traubenkernen und zur Herstellung von kaltgepresstem Traubenkernöl und Kernschrot, und
Fig. 2 eine Draufsicht auf die Vorrichtung gemäß Fig. 1.

In dem erfindungsgemäßen Verfahren (1) erfolgt das Trocknen vorzugsweise im Luftstrom bei einer Lufttemperatur von 50 bis 70 °C, vorzugsweise bei 55 bis 60 °C (das Kerngut wird dabei nicht merklich erwärmt). Durch diese Trocknung wird noch an den Kernen haftendes Gewebematerial des Tresters getrocknet und durch den Luftstrom von den Kernen getrennt. Der gewählte Temperaturbereich hat sich dabei als vorteilhaft für den Gehalt an Polyphenolverbindungen in dem Endprodukt (in dem gepressten Öl wie auch im Kernschrot) erwiesen.

Das Sieben gemäß der vorliegenden Erfindung umfasst vorzugsweise mehrere Siebschritte bzw. die Verwendung von handelsüblichen Siebmaschinen (wie Getreidesiebmaschinen), die mehrere Siebdecks mit unterschiedlicher Lochung aufweisen. Darüber hinaus kann eine solche Siebvorrichtung mit einer Absauganlage ausgerüstet sein, um durch Absaugen die leichten Teile im Kerngut (Traubenhäute, getrocknetes Gewebematerial) abzusaugen. Es werden mehrere Dauermagneten in der Reinigungsstrecke eingesetzt, um gegebenenfalls vorhandene Metallreste aus dem Kerngut zu entfernen. Durch die Reinigung sollte eine Reinheit des Kernguts von größer als 97 %, vorzugsweise größer als 99 % (bezogen auf das Gewicht des zu pressenden Kernguts) erzielt werden, da Verunreinigungen die Zersetzung der Wirksubstanzen zur Folge haben bzw. die Ausbeute an erzieltem Öl reduzieren.

Das Pressen des Verfahrens (2) erfolgt, wie vorstehend ausgeführt, bei einem Pressdruck im Presskopf (gemessen als Axiallast der Pressspindel) von 50 bis 100 kN, vorzugsweise bei einem Pressdruck von kleiner als 80 kN, jedoch so, dass die Presstemperatur des Kernschrots im Seiherkorb 50 - 80 °C, vorzugsweise 50 bis 60 °C beträgt (bei Spindelpressen wird diese Presstemperatur vor der Düse des Presskopfs bestimmt). Neben dem Pressdruck hängt die Presstemperatur dabei auch noch von dem Feuchtigkeitsgrad und dem jeweiligen Kerngut ab. Für einen möglichst hohen Gehalt an Polyphenolen in dem kaltgepressten Traubenkernöl ist es jedoch erforderlich, dass die Presstemperatur möglichst niedrig ist, jedoch keinesfalls über 80 °C liegt. Abhängig von der Presstemperatur ist ebenfalls die Temperatur des austretenden Öls, die in dem erfindungsgemäßen Verfahren beim Austritt am Seiherkorb weniger als 50 °C, vorzugsweise weniger als 40 °C betragen sollte. Das austretende Öl kühlt dann in dem Sedimentationsbehälter (siehe unten) innerhalb weniger Sekunden auf Temperaturen von unter 30 °C ab.

Weiterhin wurde gefunden, dass durch normales Filtern der Gehalt an Polyphenolverbindungen in dem gepressten Öl reduziert wird. Gemäß der vorliegenden Erfindung ist es daher bevorzugt, dass das kaltgepresste Öl in einem zusätzlichen Sedimentationsschritt von Trubstoffen befreit wird. Dieser Sedimentationsschritt findet vorzugsweise in lichtundurchlässigen Behältern (vorzugsweise aus Edelstahl) statt. Sie finden vorzugsweise in geschlossenen Behältern statt, um weitgehend Sauerstoffausschluß zu gewährleisten.

In dem Verfahren (3) der vorliegenden Erfindung erfolgen die Schritte (e) und (f) bei einer Temperatur von weniger als 40 °C und besonders bevorzugt weniger als 25 °C. In einer bevorzugten Ausführungsform des Verfahrens liegt in Schritt (e) das Verhältnis von Kernschrot zu Traubenkernöl im Bereich von 5 bis 40 Gew.-% - vorzugsweise 15 bis 25 Gew.-% -, erfolgt das Stehenlassen im lichtundurchlässigen Behältern und/oder ist die Dauer des Extraktionsvorgangs 10 bis 120 Tage, vorzugsweise 30 bis 60 Tage. Das Abtrennen in Schritt (f) erfolgt dabei wie in dem Verfahren (2) beschrieben.

Die vorliegende Erfindung betrifft weiterhin Nahrungsmittel, Nahrungsergänzungsmittel, Tiernahrungsmittel und Arzneimittel, die ein Gemisch aus kaltgepresstem Traubenkernöl und Kernschrot enthalten, das gemäß dem vorstehend beschriebenen Verfahren hergestellt wurde. Der dabei verwendete Kernschrot stammt aus dem erfindungsgemäßen Verfahren ( Pressrückstand oder Presskuchen). Es kann auch Kernschrot sein, der durch normales "Schroten" von Traubenkernen erhalten wurde. Nahrungsmittel/Tiernahrungsmittel im Sinne der vorliegenden Erfindung sind dabei Nahrungsmittel/Tiernahrungsmittel im herkömmlichen Sinne, die mit dem Gemisch aus KTK und KS versetzt wurden. Nahrungsergänzungsmittel ("Nahrungsergänzungsfaktor") sind Supplemente (z. B. in Form von Kapseln oder Tabletten) für Mensch und Tier, die zu einem wesentlichen Teil aus dem Gemisch aus KTK und KS bestehen. Das Gewichtsverhältnis von KTK zu KS in dem Nahrungsmittel, dem Nahrungsergänzungsmittel, Tiernahrungsmittel und dem Arzneimittel der vorliegenden Erfindung liegt vorzugsweise im Bereich von 30 : 70 bis 70 : 30 und insbesondere im Bereich von 40 : 60 bis 60 : 40.

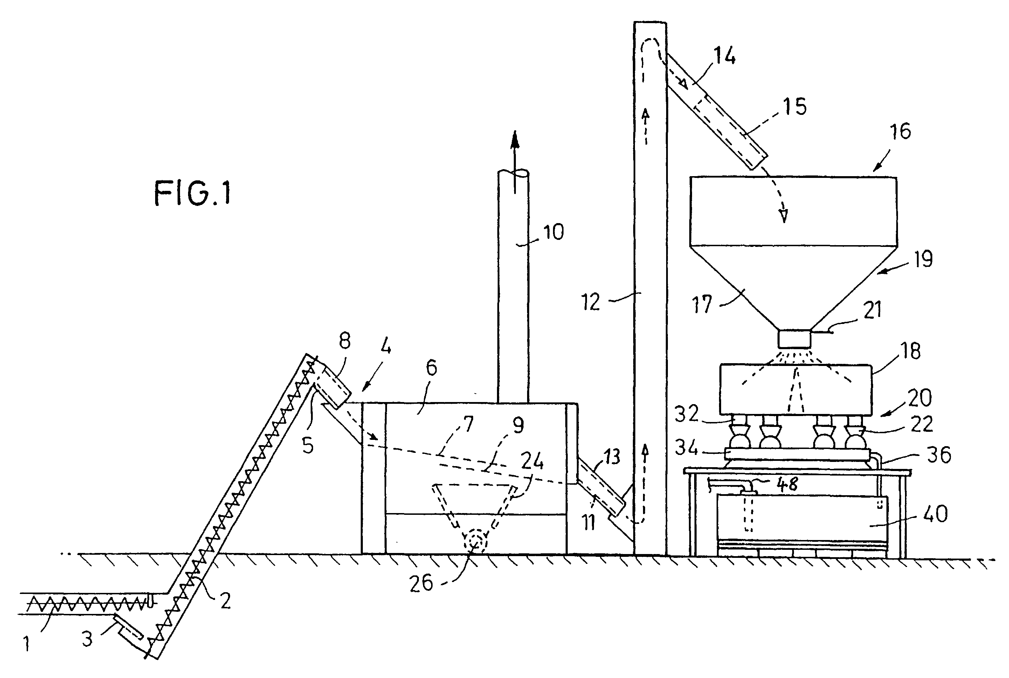

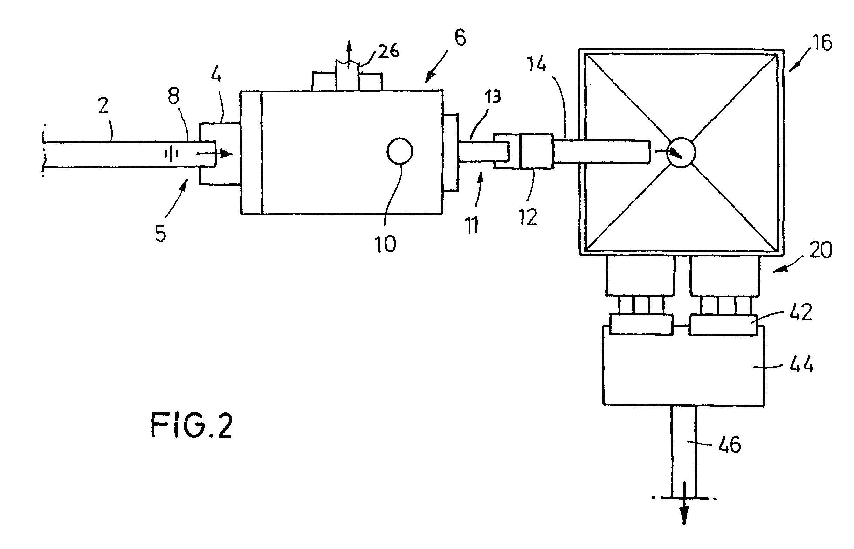

Die vorstehend beschriebenen Verfahren können vorzugsweise mittels einer in Fig. 1 und 2 gezeigten Vorrichtung mit einer ersten Fördereinrichtung (1, 2), die Traubenkerne enthaltendes Kerngut einer Siebeinrichtung (6) zuführt, die die Traubenkerne vom Ausputz, Metallteilen und Staub trennt, einer zweiten Fördereinrichtung (12) zum Transport der Traubenkerne von der Siebeinrichtung (6) zu mindestens einer Ölpresseinrichtung (20) mit einem Beschikkersilo (16), wobei Traubenkernöl einer Ölauffangwanne (34) und Kernschrot einer Kernschrotauffangwanne (44) zugeführt wird, erfolgen.

Bei der Vorrichtung sind bevorzugt mehrere Magnetabscheider (3, 5, 11, 15) einlassseitig und/oder auslassseitig an der Siebeinrichtung (6) und/oder der ersten und zweiten Fördereinrichtung (1, 2, 12) angeordnet. Weiterhin bevorzugt ist, dass eine Trocknungseinrichtung vor der Siebeinrichtung (6) angeordnet ist.

Bei der Vorrichtung ist vorzugsweise weiterhin mindestens eine Sedimentationseinrichtung (40) hinter der Ölauffangwanne (34) angeordnet. Die vorstehend erwähnte Siebeinrichtung (6) weist vorzugsweise eine Windsichteinrichtung (10) auf, die staubartige Bestandteile des Kerngutes entsorgt. Hinter der Ölpresseinrichtung (20) kann eine Pelletiereinrichtung (42) für Kernschrot angeordnet sein.

Die in dem erfindungsgemäßen Verfahren durch Sedimentation abgetrennten Trubstoffe (nachfolgend auch "Trub") haben eine wachsartige, pastöse Konsistenz und weisen einen sehr hohen Gehalt an Polyphenolverbindungen auf. In einer besonderen Ausführungsform enthält das erfindungsgemäße Nahrungsmittel, Nahrungsergänzungsmittel, Tiernahrungsmittel und Arzneimittel in allen bekannten Darreichungsformen ebenfalls noch einen gewissen Anteil an diesem Trub. Bevorzugt ist dabei, dass das Arzneimittel/Nahrungsmittel/Nahrungsergänzungsmittel/Tiernahrungsmittel jeweils mindestens 10 Gew.-%, besonders bevorzugt mindestens 30 Gew.-%, an kaltgepresstem Traubenkernöl, Kernschrot und Trub enthält.

Die erfindungsgemäßen Nahrungsmittel, Getränke, Genußmittel, Nahrungsergänzungsmittel, Tiernahrungsmittel und Arzneimittel können weiterhin handelsübliche Mineral-, Träger- und Zusatzstoffe sowie weitere Nahrungs- und Arzneistoffe enthalten. Bevorzugt ist der Zusatz von physiologisch verträglichen Mineralsalzen, so z. B. der in der DE-A-41 27 469.5 beschriebenen Lithiumsalze wie Lithiumchlorid und Lithiumcarbonat.

Das Nahrungsergänzungsmittel/Arzneimittel kann je nach Konsistenz (die von dem Ölanteil des Gemisches und der Beschaffenheit der Zusatzstoffe abhängt) gegebenenfalls mit einer nahrungsmittel-/arzneimittelgerechten Beschichtung (Coating) versehen sein, um die orale Applikation zu erleichtern.

Eine weitere bevorzugte Applikationsform des erfindungsgemäßen Arzneimittels ist die kutane Applikation, insbesondere zur Behandlung von Hauterkrankungen. Hierbei liegt das erfindungsgemäße Arzneimittel in Salbenform oder in Form eines Pflasters vor, das mit dem erfindungsgemäßen Gemisch aus KTK und KS versehen ist. Solch ein Pflaster eignet sich insbesondere zur Behandlung von Hauterkrankungen wie Psoriasis, Verbrennungen, Hautirritationen wie Sonnenbrand und Insektenstichen und kleinerer Hautverletzungen wie Schnitte und Hautabschürfungen. Die vorliegende Erfindung betrifft daher ebenfalls ein Verfahren zur Behandlung von Hauterkrankungen, umfassend das Auftragen des vorstehend beschriebenen Gemisches aus KTK und KS auf den befallenen Hautbereich. Das vorstehend beschriebene Gemisch aus KTK und KS kann ebenfalls Bestandteil von Kosmetika wie Hautpflegemittel und Sonnenschutzmittel sein.

Ebenso kann die erfindungsgemäße Extraktion der Wirkstoffe aus dem Kernschrot - insbesondere der cyclischen Polyphenole - insbesondere Ölen, in Wein und anderen Getränken und Flüssigkeiten im Rahmen einer gesundheitsorientierten Ernährung sowie in der Nahrungsergänzung, Hautpflege, Kosmetik und Medizin durch nachhaltige Verbesserung des Wirkstoffgehaltes die gesundheitliche Wirksamkeit der genannten Mittel deutlich erhöhen.

Die Wirkung des erfindungsgemäßen Nahrungsmittels bzw. Arzneimittels ergibt sich aus der Tatsache, dass die cyclischen Polyphenole verschiedenen Typus - die durch die schonende Behandlung bzw. Anreicherung um einen Faktor 100 bis 1000 höher in dem erfindungsgemäßen KTK und KS vorhanden sind - besonders wichtige gesundheitsfördernde Eigenschaften besitzen:
- Senkung des Blutalkoholspiegels durch Resorptionsminderung,
- Hemmung der PDGF- und NF-Faktoren bei der Entstehung von Herzkreislauferkrankungen in Ergänzung zusätzlicher antioxidativer Eigenschaften,
- Hemmung des Zellwachstums über die wichtigen "early genes" (c-myc- und c-fos-Proteine) im Nucleolus der Zellkerne, die der Synthese von DANN dienen,
- Hemmung von Krebswachstum (Magen/Speiseröhre/Lunge) (s. GAO et al., 1994, J. Natl. Cancer Inst. 86:855, 1994),
- detoxifizierende Enzymaktivierung und Aktivierung des programmierten Zelltodes (Apoptose) (Levitzki et al., Science 1995, 267:1782),
- Hemmung onkogener Genexpressionen (Proto-Onkogen-Produktion) (Sanchinidis et al., Mol Biol of the Cell 1999, 10:1093),
- Hemmung durch spezifische Proteinkinasen der TNF-α und IL-1-Synthesen, die vom Bakterientoxin, dem LPS (Lipopolysaccharid) über die Tyrosinkinasen inhibiert wird (Weinstein et al., Natl., Acad. Sci. USA 1991, 88:4148) und dadurch bei de Sepsis und rheumatischen Arthritis beeinflussen könnte und
- lange bekannter antioxidativer Zusatzeffekt, der größer als der des Vitamin C ist und das Vitamin E übertrifft und als "Radikalenfänger" allgemein gültig akzeptiert wird.

Schließlich wurde gefunden, dass der bei dem erfindungsgemäßen Verfahren anfallende Kernschrot ein ausgezeichneter Dünger für Pflanzen (sowohl Blumen als auch Gemüse) darstellt. Der Kernschrot kann dabei mit oder ohne zusätzliche Düngestoffe wie Mineralsalze und Bindemittel eingesetzt werden. Geeignete Mineralsalze sind z. B. die vorstehend genannten Lithiumsalze.

Die vorliegende Erfindung wird anhand des folgenden Beispiels näher erläutert:

### Beispiel

1. Pressen: Das Pressen erfolgt mittels einer in Fig. 1 und 2 abgebildeten Vorrichtung.

1.1. Fördern zur Siebmaschine: Das Kerngut wird durch eine erste und zweite Förderschnecke 1, 2 in den Annahmetrichter 4 der Siebmaschine 6 gefördert. Zwischen der ersten und der zweiten Förderschnecke 1,2 ist eine erste Dauermagneteinrichtung 3 zum Abscheiden von Metallteilen angeordnet. Am Ende der Förderschnecke 2 fällt das Kerngut durch ein etwa 1 m langes Rohr 8 in den Annahmetrichter 4. 50 cm über dem Auslauf dieses Rohres 8 in den Annahmetrichter befindet sich ein Sensor, der immer dann die Förderung sowohl der ersten Förderschnecke 1 als auch der zweiten Förderschnecke 2 ausschaltet, wenn das Kerngut den Annahmeschacht 4 der Siebmaschine 6 gefüllt hat und dann bis zur Höhe des Sensors im Rohr 8 hochsteigt.

An dem zu dem Annahmetrichter 4 führenden Rohr 8 der Siebmaschine (6) befindet sich eine zweite Dauermagneteinrichtung 5 , die die Aufgabe hat, Metallteile (Drahtstückchen, Nägel etc.) aus dem Kerngut auszusondern.

1.2. Siebmaschine: Als Siebmaschine 6 wird eine handelsübliche Getreidesiebmaschine verwendet. Sie weist zwei Siebeinrichtungen auf, wobei ein oberes Siebblech 7 eine Lochung von 6 mm Durchmesser hat, und ein unteres Siebblech 9 eine Lochung von 3 mm. Weiterhin ist die Siebmaschine 6 mit einer Absauganlage 10 für leichte Teile im Kerngut (Traubenhäute) ausgerüstet, die auch den ausgesiebten Staub über Dach oder seitlich ausbläst (sehr geringer Staubanfall).

Das obere und das untere Siebblech 7, 9 sind in Richtung auf ein Ausgangsrohr 13 der Siebmaschine 6 geneigt. Auf dem unteren Siebblech 9 sammeln sich die zur Pressung vorgesehenen Traubenkerne und beispielsweise mit Hilfe der Rüttel- oder Vibrationseinrichtung zum Ausgangsrohr 13 befördert.

Am Ausgangsrohr 13 der Siebmaschine 6 ist eine weitere Magneteinrichtung 11 angebracht. Mit dem Ausgangsrohr 13 erfolgt die Übergabe der gesiebten Traubenkerne in einen Elevator 12.

Die Siebmaschine 6 hat drei Abgabeschächte für den Ausputz (ausgesiebtes Material):
a) Grobteile, die auf der Oberfläche des ersten Siebblechs 7 (Hochsiebes) abgerüttelt werden. Diese Teile (Steinchen, Holzstückchen etc.) werden als Müll entsorgt. Erfahrungsgemäß fällt hier nur eine äußerst geringe Menge an, die in einen Stoffsack entsorgt werden kann.
b) Der Ausputz von zwei weiteren Ausgabeschächte wird in einer Auffangwanne 24 mit einer kleinen Förderschnecke 26 gesammelt. Die Förderschnecke 26 wird durch einen Sensor gesteuert, der im oberen Teil der Auffangwanne etwa 20 cm unter dem oberen Rand angebracht wird und jeweils die Förderschnecke 26 dann in Betrieb setzt, wenn der Ausputz-Schüttkegel diesen Sensor erreicht hat.
c) Das ausgesiebte Material wird von der Schnecke auf eine weitere Schnecke übergeben, von dieser auf einen Abwurfhaufen gefördert.
d) Um für diese zweite Schnecke (Schrägförderung) ausreichend Bodenfreiheit zu haben, ist auf eine entsprechende Montagehöhe der Siebmaschine 6 zu achten. Notwendig sind etwa zusätzlich 50 cm Bodenfreiheit, die durch Unterlegen der Stützfüße der Siebmaschine 6 mittels Leimbindern erreicht werden kann.

1.3. Beschicken: Das endgereinigte Kernmaterial wird über den Elevator 12 und ein am Elevator anschließendes Fallrohr 14 an dessen Ende sich wiederum ein Festmagnet 15 befindet (letzte Magnetstation zur Aussiebung etwaiger metallischer Restteile), in das Beschickersilo 16 übergeben.

Der Auslauf des Fallrohrs 14 wird so hoch angebracht, dass sich über dem Beschikkersilo 16 ein Schüttkegel bilden kann, um die volle Lagerkapazität dieses Silos ausnutzen zu können.

Das Beschickersilo 16 steht über Ölpresseinrichtungen 20, so dass die Kerne im natürlichen Gefälle in den Verteilerkasten 18 unmittelbar zu den Aufnahmetrichtern 22 der Ölpressen gelangen können. Es hat sich in der Praxis herausgestellt, dass der Winkel der Tirchterwände 17 des Trichters 19 am Boden des Beschickersilos 16 55° sein sollte, um ein glattes Rutschen der Kerne zu ermöglichen. Bei geringerem Winkel, z. B. 44°, kann es vorkommen, dass ein Teil der Kerne auf den schrägen Trichterwänden 17 liegen bleibt.

Die Kerne rutschen durch das per Schieber 21 schließbare Auslaufrohr 30 des Beschickersilos 16 in den Verteilertrog 18, der mit seinen vier Fallrohren 32 (Plastikrohre von 100 mm Durchmesser und etwa 150 mm Länge) in die Aufnahmetrichter 22 der vier Ölpresseinrichtungen 20 hineinragt. Die Ölpresseinrichtungen 20 bestehen aus Spindelpressen.

Da die Kerne in den Aufnahmetrichtern 22 wiederum kleine Schüttkegel bilden, wird der Fluss des Kernmaterials automatisch geregelt, und eine Gefahr des Überfließens der Aufnahmetrichter 22 ist auch dann nicht gegeben, wenn der Auslauf der senkrechten Fallrohre 32 des Verteilertrogs 18 in einer Höhe mit der Kante der Aufnahmetrichter 22 liegt.

1.4. Pressen: Eingesetzt werden zwei Komet-Doppelschneckenpressen vom Typ DD85G mit vier modifizierten Pressschnecken vom Typ R8 und modifizierten Düsen mit einem Durchmesser von 15 mm.

Ölausbeute und Ölqualität werden von zwei Faktoren bestimmt:
a) Reinheit des Pressguts (Kerne)
   Je reiner das Kerngut (also frei von Traubenhäuten und Stengelchen etc.), um so besser ist die Ölqualität und um so besser auch die Ausbeute, da die Fremdstoffe für höheren Trubanteil sorgen, der ähnlich wie ein Schwamm das gepresste Öl aufnimmt.
b) Pressdruck
   Der Pressdruck entscheidet über die Ölqualität (Presstemperatur), ebenso über die Höhe der Ausbeute.

Der Pressdruck wird beeinflusst durch die Steigung der Spindel, durch die Geschwindigkeit (Umdrehungszahl) und durch den Düsenquerschnitt (je größer die Düsenöffnung, um so geringer der Pressdruck).

In der durch praktische Versuche erarbeiteten optimalen Konstellation dieser Faktoren haben sich die oben erwähnten modifizierten Pressspindeln und Düsen bewährt, ebenso eine über die Reibkupplung einzustellende Umdrehungsgeschwindigkeit der Spindeln, die auf der Einstellskala bei dem Wert 3,5 liegt.

1.5. Pelletieren: Die ausgepressten Kerne treten als Strang mit einem Durchmesser, der etwa der Düsenöffnung entspricht (15 mm) aus und würden ohne weitere mechanische Bearbeitung in unregelmäßigen Strangabschnitten von 10 bis 80 cm abbrechen. Im Transport des Kernschrots würden mit diesen Strängen größere Hohlräume entstehen, so dass die Gesamtladung geringer angesetzt werden müßte. Ebenso sind die Stränge in der Weiterverarbeitung z. B. in Schrotmühlen weniger geeignet.

Aus diesem Grund werden die Stränge auf Abschnitte von etwa 3 cm Länge pelletiert. Dazu wurde eine Pelletiereinrichtung 42 konstruiert, die mit einem stark untersetzten Getriebe (niedrige Umdrehungszahl), das von einem kleinen Elektromotor angetrieben wird, mit Hilfe eines stumpfen Schlagmessers die Stränge unmittelbar nach dem Austreten aus den Düsen der Ölpresseinrichtungen 20 abschneidet. Die Länge der Strangabschnitte ist einstellbar, indem die Pelletiereinrichtung 42 auf zwei Schienen, die an den Ölpresseinrichtungen 20 montiert sind, verschoben werden kann, so dass das Schlagmesser näher oder weiter entfernt zum/vom Ende der Düsen auf den Pressstrang trifft.

In Weiterentwicklung der vorgenannten Technik wurde eine Pelletiermaschiene entwickelt, bei sich eine in ihrer Umdrehungszahl veränderbare Förderschnecke rotiert. Zwischen Rohrwandung und Schneckenrand werden die Pressstränge zu Pellets zerschnitten, deren Länge durch die Umdrehungszahl der Schnecke (regelbarer Elektromotor) bestimmbar ist. Die Schnecke dient gleichzeitig zum Herausfördern der Pellets auf eine weitere Transporteinrichtung.

Die Pellets des Kernschrots fallen über einen Auffangwanne 44 in eine Schneckenfördereinrichtung 46, die das Schrotmaterial auf einen Abwurfhaufen oder in einen Container/Silo fördern. Die Schnecke 46 ist zeitgesteuert: Etwa alle 40 min schaltet die Automatik die Schnecke 46 für 30 s ein. Diese Zeit reicht aus, um das im Auffangtrichter 44 angesammelte Kernschrotmaterial komplett abzufördern. Durch diese Zeitschaltautomatik werden gegenüber einem Dauerbetrieb der Schnecke 46 erhebliche Energiekosten (Stromverbrauch) gespart, und der Verschleiß wird niedrig gehalten.

### 2. Öl

2.1. Auffangwanne: Unter den Seiherkörben der Ölpresseinrichtungen 20 steht eine Auffangwanne 34 aus Edelstahl mit einem dreieckigen Querschnitt und einem Gefälle zum Auslauf hin. So ist gewährleistet, dass das frischgepresste Öl immer über einen Kunststoffschlauch 36 abfließt.

2.2. Trubwanne: Das Öl fließt in eine Edelstahltrubwanne 40 mit rechteckigem Querschnitt und einem Fassungsvermögen von 500 I. Die Trubwanne 40 ist abgedeckt, um eine Verunreinigung durch Staub oder Hineinfallen von Fremdteilen zu vermeiden.

Die Trubwanne 40 hat die Funktion, dem Öl über das natürliche Absetzen den Trub weitgehend zu entziehen. Folgerichtig ist der Auslauf aus der Trubwanne 40 in Form eines Rohres 48 vorzusehen, das über eine Manschette durch Befestigungsschrauben festgestellt werden kann und dadurch in der Höhe verschiebbar ist, also unterschiedlich tief in die Trubwanne 40 hineinragt. So kann durch Absaugen der obersten Ölschicht vermieden werden, dass ein größerer Trubanteil mit dem Öl in einen Container, der als weitere Stufe des Trubabsetzens vorgesehen ist, hineinfließt.

Die Ölförderung kann von einer kleinen Impellerpumpe mit 200 bis 400 W-Motor ausreichend bewältigt werden (Schlauchmaterial: Kunststoff).

Um ein gefiltertes, aber klares Öl zu erhalten, das in dieser Form marktfähig ist, muß das Öl nach dem Umpumpen von der Trubwanne 40 in den ersten Container mindestens noch einmal umgepumpt werden, besser zweimal, so dass es erst aus einem dritten Container zur Abfüllung kommt. Da das kaltgepresste Traubenkernöl zwar weder hitze- noch kälteempfindlich und auch gegen die Oxidation weitgehend resistent, jedoch stark lichtempfindlich ist, sollten die Ölcontainer ständig mit schwarzer Folie abgedeckt werden.

2.3. Filtration: Bei dem oben beschriebenen Trubabsetzverfahren bleibt ein Öltrubrest von etwa 20 % der Gesamtmenge. Dieses Öl mit einem hohen Trubanteil muß über Tuchfilter (Kammerdruckpressen) filtriert werden. Dazu ist jede Filterpresse dieses Typs geeignet.

Zur Unterstützung der Filtrationswirkung wird Kieselgur in das zu filtrierende Öl (Öltrubmischung) eingerührt.

Das so erzeugte Öl ist noch klarer und brillanter als das unfiltrierte, hat jedoch einen etwas geringeren Anteil an Wertstoffen und Geschmacksstoffen.

## Patentansprüche

1. Verfahren zum Aufreinigen von Traubenkernen, umfassend die Schritte
(a) schonendes Trocknen der Traubenkerne im Luftstrom bei 50 bis 70 °C Lufttemperatur und
(b) Reinigen der getrockneten Traubenkerne, das Sieben und Abtrennung von Metallteilen mittels Dauermagneten einschließt.

2. Verfahren nach Anspruch 1, wobei das Trocknen im Luftstrom bei 55 bis 60 °C Lufttemperatur erfolgt.

3. Verfahren nach Anspruch 1 oder 2, wobei das Sieben mehrere Siebschritte umfasst und durch die Reinigung ein Reinheitsgrad des Kernguts von größer als 97 %, vorzugsweise größer als 99 % erzielt wird.

4. Verfahren zur Herstellung von kaltgepresstem Traubenkernöl und Kernschrot, umfassend Trocknen und Reinigen nach einem oder mehreren der Ansprüche 1 bis 3 und
(c) Pressen der gereinigten Traubenkerne bei einem Pressdruck im Presskopf von 50 bis 100 kN unter Erhalt von kaltgepresstem Traubenkernöl und Kernschrot.

5. Verfahren nach Anspruch 4, wobei der Pressdruck im Presskopf kleiner als 80 kN ist und/oder der Pressdruck so eingestellt ist, dass die Presstemperatur des Kernschrots im Seiherkorb 50 bis 80 °C, vorzugsweise 50 bis 60 °C, beträgt.

6. Verfahren nach Anspruch 4 oder 5, wobei die Temperatur des auslaufenden Öls weniger als 50 °C, vorzugsweise 40 °C, beträgt.

7. Verfahren nach einem oder mehreren der Ansprüche 4 bis 6, weiterhin umfassend die Schritte
(d) Extraktion von Inhaltstoffen aus dem gemäß Ansprüchen 4 bis 6 erhältlichen Kernschrot durch Versetzen des Kernschrots mit dem gemäß Ansprüchen 4 bis 6 erhältlichen kaltgepressten Traubenkernöl, ebenso aber auch mit anderen Speiseölen
(e) Stehenlassen des in Schritt (d) erhältlichen Gemisches und
(f) Abtrennen des Traubenkernöls beziehungsweise eines anderen Speiseöles von dem Kernschrot.

8. Verfahren nach Anspruch 7, wobei die Schritte (e) und (f) bei einer Temperatur von weniger als 40 °C und besonders bevorzugt weniger als 25 °C erfolgen.

9. Verfahren nach Anspruch 7 oder 8, wobei in Schritt (e) das Verhältnis von Kernschrot zu Traubenkernöl im Bereich von 5 bis 40 Gew.-% - vorzugsweise 15 bis 25 Gew.-% - liegt, das Stehenlassen im lichtundurchlässigen Behältern erfolgt und/oder die Dauer des Extraktionsvorgangs 10 bis 120 Tage, vorzugsweise 30 bis 60 Tage ist.

10. Verfahren nach einem oder mehreren der Ansprüche 4 bis 9, wobei das Verfahren weiterhin mindestens einen Sedimentationsschritt (g) zum Abtrennen von Trubstoffen umfasst.

11. Verfahren nach Anspruch 7, wobei der Sedimentationsschritt Stehenlassen des gepressten Öls in lichtundurchlässigen Behältern umfasst.

12. Traubenkernöl, erhältlich nach einem oder mehreren der Ansprüche 1 bis 11, insbesondere nach einem oder mehreren der Ansprüche 1 bis 9.

13. Verfahren zur Extraktion von Inhaltsstoffen aus dem gemäß Ansprüchen 4 bis 6 erhältlichen Kernschrot, umfassend das. Versetzen des Kernschrots mit einem geeigneten Extraktionsmittel wie Ölen, Wein und anderen Getränken sowie anderen Flüssigkeiten.

14. Nahrungsmittel, Nahrungsergänzungsmittel, Nahrungsmittelzusatzfaktor, Tiernahrungsmittel, Tiernahrungsmittelergänzungsfaktor, Arzneimittel und Kosmetika in allen Darreichungsformen, umfassend
(a) das gemäß Ansprüchen 1 bis 11, insbesondere das gemäß Ansprüchen 7 bis 9 erhältliche kaltgepresste Traubenkernöl;
(b) das gemäß Ansprüchen 1 bis 6 erhältliche Kernschrot; oder
(c) ein Gemisch aus (a) und (b).

15. Nahrungsmittel und Nahrungsergänzungsmittel, enthaltend die nach Ansprüchen 1 bis 3 erhältlichen Traubenkerne und/oder Traubenkernmehl aus den genannten Traubenkernen.

16. Nahrungsmittel und Nahrungsergänzungsmittel gemäß Anspruch 14 oder 15, die Backwaren sind.

17. Nahrungsergänzungsmittel und Arzneimittel nach Anspruch 14, die ein Ergänzungsfaktor für immunsupprimierte und rekonvaleszente Kranke sind.

## Claims

1. A process for purifying grape seeds, comprising the steps of:
(a) carefully drying the grape seeds in a current of air at an air temperature of from 50 to 70 °C; and
(b) purifying the dried grape seeds, which includes the sieving and separating of metal parts using permanent magnets.

2. The process according to claim 1, wherein said drying is effected in a current of air at an air temperature of from 55 to 60 °C.

3. The process according to claim 1 or 2, wherein said sieving comprises several sieving steps and a degree of purity of the seed material of greater than 97%, preferably greater than 99%, is achieved by said purification.

4. A process for preparing cold-pressed grape seed oil and crushed seeds, comprising the drying and purifying according to one or more of claims 1 to 3; and
(c) pressing the purified grape seeds with a pressing power in the pressing head of from 50 to 100 kN to obtain cold-pressed grape seed oil and crushed seeds.

5. The process according to claim 4, wherein the pressing power in the pressing head is lower than 80 kN, and/or the pressing power is adjusted to have a pressing temperature of the crushed seeds within the strainer basket of from 50 to 80 °C, preferably from 50 to 60 °C.

6. The process according to claim 4 or 5, wherein the temperature of the outflowing oil is less than 50 °C, preferably less than 40 °C.

7. The process according to one or more of claims 4 to 6, further comprising the steps of:
(d) extracting components from the crushed seeds obtainable according to claims 4 to 6 by adding the cold-pressed grape seed oil obtainable according to claims 4 to 6 or other edible oils to the crushed seeds;
(e) allowing the mixture obtainable in step (d) to stand; and
(f) separating the grape seed oil or the other edible oil from the crushed seeds.

8. The process according to claim 7, wherein steps (e) and (f) are performed at a temperature of less than 40 °C, preferably less than 25 °C.

9. The process according to claim 7 or 8, wherein the ratio of crushed seeds to grape seed oil in step (e) is within a range of from 5 to 40% by weight, preferably from 15 to 25% by weight; said allowing the mixture to stand is done in lightproof tanks; and/or the duration of the extraction process is from 10 to 120 days, preferably from 30 to 60 days.

10. The process according to one or more of claims 4 to 9, wherein said process further comprises at least one sedimentation step (g) for separating turbid matters.

11. The process according to claim 7, wherein the sedimentation step comprises allowing the pressed oil to stand in lightproof containers.

12. A grape seed oil obtainable according to one or more of claims 1 to 11, especially according to one or more of claims 1 to 9.

13. A process for extracting components from the crushed seeds obtainable according to claims 4 to 6, comprising the addition of a suitable extractant, such as oils, wine and other beverages as well as other fluids, to the crushed seeds.

14. A food, food supplement, food additive, animal food, animal food supplement, medicament or cosmetic in all dosage forms, comprising:
(a) the cold-pressed grape seed oil obtainable according to claims 1 to 11, especially claims 7 to 9;
(b) the crushed seeds obtainable according to claims 1 to 6; or
(c) a mixture of (a) and (b).

15. A food or food supplement containing the grape seeds obtainable according to claims 1 to 3 and/or grape seed meal obtainable from said grape seeds.

16. The food or food supplement according to claim 14 or 15 which is pastries.

17. The food supplement or medicament according to claim 14 which is a supplement for immunosuppressed and convalescent patients.

## Revendications

1. Procédé de nettoyage de pépins de raisin, comprenant les étapes consistant:
(a) à sécher avec ménagement les pépins de raisin dans un courant d'air, à une température d'air comprise entre 50° et 70°C, et
(b) à nettoyer les pépins de raisin séchés, ce nettoyage incluant le tamisage et la séparation des parties métalliques en utilisant des aimants permanents.

2. Procédé selon la revendication 1, dans lequel le séchage s'effectue dans un courant d'air à une température d'air de 55 à 60°C.

3. Procédé selon la revendication 1 ou 2, dans lequel le tamisage comprend plusieurs étapes de tamisage et dans lequel un degré de pureté du produit en pépins, supérieur à 97 %, de préférence supérieur à 99 %, est obtenu par le nettoyage.

4. Procédé de fabrication d'une huile de pépins de raisins pressée à froid et de tourteau de pépins, comprenant le séchage et le nettoyage selon ou plusieurs des revendications 1 à 3, et
(c) le pressage des pépins de raisins nettoyés, à une pression de pressage, dans la tête de pressage, de 50 à 100 kN en obtenant une huile de pépins de raisins pressée à froid et du tourteau de pépins.

5. Procédé selon la revendication 4, dans lequel la pression de pressage dans la tête de pressage est inférieure à 80 kN et/ou la pression de pressage est réglée de manière que la température de pressage du tourteau de pépins, dans le panier de filtration, soit de 50 à 80°C, de préférence de 50 à 60°C.

6. Procédé selon la revendication 4 ou 5, dans lequel la température de l'huile sortante est inférieure à 50°C, de préférence à 40°C.

7. Procédé selon l'une ou plusieurs des revendications 4 à 6, comprenant en outre les étapes consistant :
(d) à extraire des substances contenues à partir du tourteau de pépins obtenu selon les revendications 4 à 6, par mélange du tourteau de pépins avec l'huile de pépins de raisins pressée à froid, obtenue selon les revendications 4 à 6, de même, également, d'autres huiles alimentaires ou comestibles,
(e) à laisser reposer le mélange obtenu à l'étape (d), et
(f) à séparer l'huile de pépins de raisins respectivement d'une autre huile alimentaire du tourteau de pépins.

8. Procédé selon la revendication 7, dans lequel les étapes (e) et (f) sont effectuées à une température inférieure à 40°C, en particulier, de préférence, inférieure à 25°C.

9. Procédé selon la revendication 7 ou 8, dans lequel, à l'étape (e), le rapport entre le tourteau de pépins et l'huile de pépins de raisins est dans la plage de 5 à 40 % en poids - de préférence de 15 à 25 % en poids - dans lequel la phase de repos s'effectue dans des récipients non transparents à la lumière et/ou la durée du processus d'extraction est de 10 à 120 jours, de préférence de 30 à 60 jours.

10. Procédé selon l'une ou plusieurs des revendications 4 à 9, dans lequel le procédé comprend en outre au moins une étape de sédimentation (g) pour la séparation des substances qui se déposent.

11. Procédé selon la revendication 7, dans lequel l'étape de sédimentation comprend le maintien au repos de l'huile pressée, dans des récipients non transparents à la lumière.

12. Huile de pépins de raisins, obtenue selon l'une ou plusieurs de revendications 1 à 11, en particulier selon l'une ou plusieurs des revendications 1 à 9.

13. Procédé d'extraction des substances contenues dans du tourteau de pépins obtenus selon les revendications 4 à 6, comprenant le mélange du tourteau de pépins avec un agent d'extraction approprié, tel que des huiles, du vin et d'autres boissons, ainsi que d'autres liquides.

14. Produit alimentaire, produit de complément alimentaire, facteur additif d'un produit alimentaire, produit d'alimentation animale, facteur de complément de produit d'alimentation animale, produit pharmaceutique et cosmétique, sous toutes les formes d'administration, comprenant :
(a) l'huile de pépins de raisins pressée à froid, obtenue selon les revendications 1 à 11, en particulier selon les revendications 7 à 9
(b) le tourteau de pépins obtenu selon les revendications 1 à 6 ; ou
(c) un mélange de (a) et (b).

15. Produit alimentaire et produit de complément alimentaire, contenant les pépins de raisins et/ou la farine de pépins de raisins obtenus selon les revendications 1 à 3 à partir des pépins de raisins cités.

16. Produit alimentaire et produit de complément alimentaire selon la revendication 14 ou 15, qui sont des produits agglomérés et cuits.

17. Produit de complément alimentaire et produit pharmaceutique selon la revendication 14 qui sont un facteur de complément pour des malades à immunité déficiente et reconvalescents.
